# EUROPEAN PATENT APPLICATION

(11) **EP 2 199 274 A1**
(43) Date of publication of application: **23.06.2010**
(21) Application number: 08384013.2
(22) Date of filing: 16.12.2008
(51) Int. Cl.: C07C 217/74, C07C 233/25, A61K 31/135, A61K 31/167, A61P 25/00

(54) **Co-crystals of tramadol and paracetamol**

(71) Applicant: Laboratorios Del. Dr. Esteve, S.A., 08041 Barcelona (ES)
(72) Inventor: Dr. Buschmann, Helmut Heinrich, 52076 Aachen (Walheim) (DE); Tesson, Nicolas, 08028 Barcelona (ES); Farran, Joan, 08028 Barcelona (ES)
(74) Representative: Peters, Hajo

(57) **Abstract**

The present invention relates to co-crystals of tramadol and paracetamol, processes for preparation of the same and their uses as medicaments or in pharmaceutical formulations, more particularly for the treatment of pain.

## Description

The present invention relates to co-crystals of tramadol and paracetamol, processes for preparation of the same and their uses as medicaments or in pharmaceutical formulations, more particularly for the treatment of pain.

Pain is a complex response that has been functionally categorized into sensory, autonomic, motor, and affective components. The sensory aspect includes information about stimulus location and intensity while the adaptive component may be considered to be the activation of endogenous pain modulation and motor planning for escape responses. The affective component appears to include evaluation of pain unpleasantness and stimulus threat as well as negative emotions triggered by memory and context of the painful stimulus.

In general, pain conditions can be divided into chronic and acute. Chronic pain includes neuropathic pain and chronic inflammatory pain, for example arthritis, or pain of unknown origin, such as fibromyalgia. Acute pain usually follows non-neural tissue injury, for example tissue damage from surgery or inflammation, or migraine.

There are many drugs that are known to be useful in the treatment or management of pain. Opioids are frequently used as analgesics in pain. Derivatives of morphine are indicated for the treatment of moderate to acute pain in human. The analgesic effect is obtained through their action on morphinic receptors, preferably the µ-receptors. Among these derivatives of morphine, may be mentioned morphine, codeine, pethidine, dextropropoxyphenemethadone, lenefopan and others.

One of the morphinic derivatives that has shown very good results when orally administered, and which is extensively marketed, is tramadol, also available as a physiologically acceptable salt, particularly as a hydrochloride. Tramadol, whose chemical name is 2-(dimethylaminomethyl)-1-(3-methoxyphenyl)cyclohexanol, has the following formula :

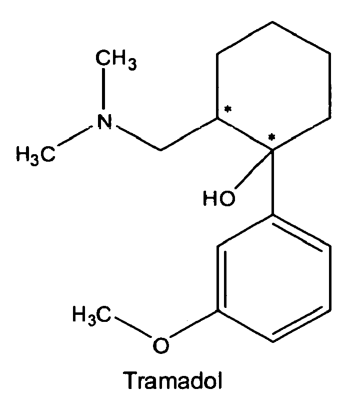

This structure shows two different chiral centers and thus may exist in different diastereoisomers among which the tramadol is the cis-diastereoisomer : (1R, 2R), or (1S, 2S), both also known as (+)-tramadol and (-)-tramadol and both of which contribute in different ways to its activity.

From the art it appears that this compound is neither fully opioid-like, nor non-opioid-like. Some studies have demonstrate that tramadol is an opioid agonist, whereas clinical experience indicates that it lacks many of the typical side effects of opioid agonists, for example respiratory depression, constipation or tolerance.

Due to their drawbacks, opioids cannot always be given repeatedly or at higher doses as analgesics to treat pain. The side effects of opioids are known in the art including e.g. J. Jaffe in "Goodman and Gilman's, The Pharmacological Basis of Therapeutics", 8th edition; Gilman et al.; Pergamon Press, New York, 1990, Chapter 22, pages 522-573.

Consequently it has been proposed to combine opioids with other drugs that are not opioid analgesic agents, in order to lower the amount of opioids needed to produce an equivalent degree of analgesia.

Thus it was the objective of the current invention to provide new means of improving the properties of tramadol, especially in regard to the treatment of pain, by providing new drugable forms of tramadol.

Especially desirable improvements/advantages of the new drugable form would include any of the following:
- improvement of physicochemical properties in order to facilitate the formulation, the manufacture, or to enhance the absorption and/or the bioavailability:
thus
- being more active when compared to tramadol base or hydrochloride salt;
- providing a form of tramadol with a further active agent having a beneficial pharmacological effect in itself, thus allowing for a highly efficient dose/weight relation of the final active principle; or even
- allowing the use of a lower therapeutic dose of either tramadol and the further active agent paracetamol, or of both;
- having a synergistic effect through the combination of tramadol and the further active agent paracetamol, in the same new drugable form; or further
- having the bitter taste of tramadol removed or ameliorated;
- being easily obtainable, easy to manufacture;
- allowing more flexibility in formulating, or facilitating its formulation,
- being highly soluble, thus allowing better dissolution rates, especially if dissolving in an aqueous physiological surrounding;
- improving stability of the co-crystal in comparison to the physical mixture of tramadol/paracetamol at the same ratio; or
- allowing new routes of administration;
also
- minimizing/reducing the side effects, especially the severe side effects, assigned to tramadol.

Most desirably the new drugable forms should combine more than one, or most of these advantages.

This objective was achieved by providing new co-crystals of tramadol. It was found that tramadol was able to form co-crystals with paracetamol. These co-crystals show improved properties if compared to tramadol alone, and also good analgesic activity. The co-crystals thus obtained have a specific stoichiometry. Under the proper circumstance this is also another advantage of these new solid drugable forms possibly achieving some modulation of the pharmacological effects. While APIs (Active Pharmaceutical Ingredients) like tramadol in general have been recognized to form crystalline polymorphs, solvates, hydrates and amorphous forms for a number of years, there is little knowledge about which APIs will form co-crystals. Co-crystals are a specific type of crystalline form which provide a new avenue to modulate the API form and thus to modulate API properties. Pharmaceutical co-crystals contain an API and at least one other component which crystallize together. Selection of the other component helps determine whether a co-crystal will form and what properties the co-crystal will have. Just as a polymorph, solvate, hydrate or amorphous form of an API can modulate stability, solubility, and hygroscopicity, a co-crystal can modulate those same properties.

Thus the main object of the present invention is a co-crystal comprising tramadol either as a free base or as its physiologically acceptable salt and paracetamol.

"Drugable form (of tramadol)" as used herein is defined as any form (salt, amorphous, crystal, solution, dispersion, mixture etc,) that tramadol might take which still can be formulated into a pharmaceutical formulation usable as a medicament to treat a disease or a symptom, especially pain.

"Co-crystal" as used herein is defined as a crystalline material comprising two or more compounds at ambient temperature (20 to 25°C, preferably 20°C), of which at least two are held together by weak interaction, wherein at least one of the compounds is a co-crystal former. Weak interaction is being defined as an interaction which is neither ionic nor covalent and includes for example: hydrogen bonds, van der Waals forces, and π-π interactions. Solvates of tramadol that do not further comprise a co-crystal former are not co-crystals according to the present invention. The co-crystals may however, include one or more solvate molecules in the crystalline lattice. Just for the sake of clarity the distinction between crystalline salt and a co-crystal has to be stressed here. An API bound to another compound forming a salt by means of ionic interaction can be considered as one "compound" according to the invention, but it cannot be considered as two compounds by itself.

In scientific literature there currently is some discussion on the proper use of the word co-crystal (see for example Desiraju, CrystEngComm, 2003, 5(82), 466-467 and Dunitz, CrystEngComm, 2003, 5(91), 506). A recent article by Zaworotko (Zaworotko, Crystal Growth & Design, Vol. 7, No. 1, 2007, 4-9) gives a definition of co-crystal which is in line with the definition given above.

"Co-crystal former" as used herein is defined as paracetamol with which tramadol is able to form co-crystals.

"Active agents" are APIs which show a pharmaceutical effect and thus can be identified as being pharmaceutically active. In a more narrow sense this definition is encompassing all APIs being marketed or under clinical trial for the treatment of diseases. "Active agents with analgesic activity" are APIs (Active Pharmaceutical Ingredients) which show efficacy in well-known animal models of pain and thus can be identified as being analgesics. In a more narrow sense this definition is encompassing all APIs being marketed or under clinical trial for a labelling including an indication falling under the definition of pain, including also migraine. These indications might include acute pain, chronic pain, neuropathic pain, hyperalgesia, allodynia or cancer pain, including diabetic neuropathy or diabetic peripheral neuropathy, osteoarthritis or fibromyalgia and all their subforms. An example of an "active agents with analgesic activity" is paracetamol.

"Pain" is defined by the International Association for the Study of Pain (IASP) as "an unpleasant sensory and emotional experience associated with actual or potential tissue damage, or described in terms of such damage (IASP, Classification of chronic pain, 2nd Edition, IASP Press (2002), 210). Even though pain is always subjective its causes or syndromes can be classified.

According to the IASP "allodynia" is defined as "a pain due to a stimulus which does not normally provoke pain" (IASP, Classification of chronic pain, 2nd Edition, IASP Press (2002), 210). Even though the symptoms of allodynia are most likely associated as symptoms of neuropathic pain this is not necessarily the case so that there are symptoms of allodynia not connected to neuropathic pain though rendering allodynia in some areas broader then neuropathic pain.

The IASP further draws the following difference between "allodynia", "hyperalgesia" and "hyperpathia" (IASP, Classification of chronic pain, 2nd Edition, IASP Press (2002), 212):

| | | |
|---|---|---|
| Allodynia | Lowered threshold | Stimulus and response mode differ |
| Hyperalgesia | Increased response | Stimulus and response rate are the same |
| Hyperpathia | Raised threshold; Increased response | Stimulus and response rate may be the same or different |

According to the IASP "neuropathy" is defined as "a primary lesion or dysfunction in the nervous system" (IASP, Classification of chronic pain, 2nd Edition, IASP Press (2002), 211). Neuropathic pain may have central or peripheral origin.

In a further embodiment of the co-crystal according to the invention the ratio of tramadol to paracetamol is chosen in such a way that if compared to either tramadol alone or to a comparable mixture of tramadol and paracetamol
- the solubility of the co-crystal is increased; and/or
- the dose response of the co-crystal is increased; and/or
- the efficacy of the co-crystal is increased; and/or
- the dissolution of the co-crystal is increased; and/or
- the bioavailability of the co-crystal is increased; and/or
- the stability of the co-crystal is increased; and/or
- the hygroscopicity of the co-crystal is decreased; and/or
- the form diversity of the co-crystal is decreased; and/or
- the morphology of the co-crystal is modulated.

"Mixture of tramadol and paracetamol" is defined as a mixture of paracetamol with tramadol which is only a physical mixture without any coupling forces between the compounds and thus neither includes salts nor another co-crystal.

In a further embodiment of the co-crystal according to the invention the molar ratio between tramadol and paracetamol is different from 1. This might have the advantage of allowing the development of a non-equimolar ratio between tramadol and paracetamol in a fixed dose with all the advantages of the co-crystal.

The term "salt" is to be understood as meaning any form of tramadol or paracetamol according to the invention in which this assumes an ionic form or is charged and is coupled with a counter-ion (a cation or anion) or is in solution. By this are also to be understood complexes of tramadol or paracetamol with other molecules and ions, in particular complexes which are complexed via ionic interactions. This also includes physiologically acceptable salt.

The term "solvate" according to this invention is to be understood as meaning any form of the tramadol or paracetamol in which the compound has attached to it via non-covalent binding a solvent (most likely a polar solvent) especially including hydrates and alcoholates, e.g. monohydrate.

Paracetamol being the co-crystal former with tramadol is a highly interesting drug. It is also known as acetaminophen. Its chemical name is N-(4-hydroxyphenyl)acetamide and it is also described as a physiologically acceptable salt. It has an empirical formula of C₈H₉NO₂, and a melting point of 169°C.

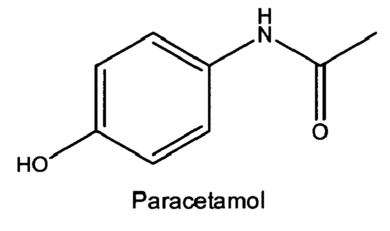

A very preferred embodiment of the invention relates to a co-crystal according to the invention, wherein the tramadol is racemic tramadol, (-)-tramadol or (+)-tramadol.

As illustrated in more detail below tramadol as its racemate and its enantiomers (+)-tramadol and (-)-tramadol form co-crystals with paracetamol. Generally the co-crystals obtained have a specific stoichiometry.

In a further preferred embodiment of the invention the co-crystal according to the invention, is selected from
- a co-crystal comprising racemic tramadol either as a free base or as its physiologically acceptable salt and paracetamol;
- a co-crystal comprising (-)-tramadol either as a free base or as its physiologically acceptable salt and paracetamol;
- a co-crystal comprising (+)-tramadol either as a free base or as its physiologically acceptable salt and paracetamol;
- an enantiomeric mixture of co-crystals comprising (-)-tramadol either as a free base or as its physiologically acceptable salt and paracetamol and co-crystals comprising (+)-tramadol either as a free base or as its physiologically acceptable salt and paracetamol; or
- any of the co-crystals above being solvate co-crystals, preferably being hydrate or alcoholate co-crystals, most preferably being monohydrate co-crystals.

In a highly preferred embodiment of these selected co-crystals the molecular ratio between tramadol and paracetamol is 1 : 1.

Another very preferred embodiment of the invention relates to a co-crystal according to the invention, showing a molecular ratio between tramadol, paracetamol and water of 1 : 1 : 1. A preferred embodiment of this relates to a co-crystal according to the invention, showing a molecular ratio between tramadol · HCl, paracetamol and water of 1 : 1 : 1.

In a preferred embodiment the co-crystal with a molecular ratio between tramadol, paracetamol and H₂O is 1 : 1 : 1 according to the invention shows a Fourier Transform Infra Red pattern with absorption bands at 3356.7 (m), 3302.8 (m), 2937.3 (m), 2678.6 (m br), 1669.18 (s), 1599.0 (m); 1553.8 (m), 1517.5 (s), 1256.7 (s), 1008.1 (m), 837.2 (m), 787.3 (m), and 702.5 (m) cm⁻¹.

In a preferred embodiment the co-crystal with a molecular ratio between tramadol, paracetamol and H₂O is 1 : 1 : 1 according to the invention has a monoclinic unit cell with the following dimensions:
a = 13.20 Å;
b = 12.17 Å;
c = 17.08 Å;
β = 110.63°.

In a preferred embodiment of the co-crystal with a molecular ratio between tramadol, paracetamol and H₂O of 1 : 1 : 1 according to the invention, the endothermic sharp peak of the co-crystal corresponding to the melting point has an onset at 74.4°C.

In a preferred embodiment of the co-crystal with a molecular ratio between tramadol, paracetamol and H₂O of 1 : 1 : 1 according to the invention, the co-crystal shows an X-ray powder diffraction pattern with peaks expressed in d-values in A at 8.52, 6.07, 5.50, 5.38, 5.25, 4.87, 4.46, 4.37, 4.32, 4.07, 3.86, and 3.34.

Another embodiment of the present invention relates to a process for the production of a co-crystal according to the invention as described above comprising the steps of:
(a) tramadol, optionally in the form of hydrochloride, and paracetamol were added to a container into which also a first non-aqueous solvent is added;
(b) adding water either with, or after, or before adding the first non-aqueous solvent from step (a) to the container;
(c) optionally adding a second non-aqueous solvent to the container;
(d) mixing the solution/dispersion of step (b) or optionally step (c) at ambient temperature;
(e) filtering-off the resulting co-crystals.

"Ambient temperature" is defined here as a temperature between 20 and 25°C, preferably being 20°C.

The first and second non-aqueous solvents usable in this process include non-aqueous organic solvents, preferably solvents selected from acetonitrile, ethyl acetate, acetone, isobutyl acetate, 2-butanol, dimethylcarbonate, chlorobenzene, butylether, diisopropylether, dimethylformamide, ethanol, water, hexane, isopropanol, methyl ethyl ketone, methanol, methyl t-butyl ether, 3 pentanone, toluene and 1,1,1-trichloroethane, most preferably solvents selected from acetonitrile, or ethyl acetate.

The molecular ratio between tramadol and paracetamol of step (a) lies between 4 : 1 to 1 : 4, preferably from 3 : 1 to 1 : 3; more preferably from 2 : 1 to 1 : 2; and most preferably is 1 : 1.

The parts of the co-crystal according to the invention are well-known drugs with analgesic properties sometimes used for a long time worldwide. Due to this a further object of the present invention is a medicament comprising a co-crystal according to the invention.

Thus the invention also concerns a medicament comprising at least one co-crystal according to the invention as described above and optionally one or more pharmaceutically acceptable excipients.

The invention also relates to a pharmaceutical composition that comprises a therapeutically effective amount of the co-crystal according to the invention in a physiologically acceptable medium.

The association of two active principles in the same crystal exhibits several advantages. Being linked, they often behave as a single chemical entity, thus facilitating the treatments, formulation, dosage etc. In addition to that, with both tramadol and paracetamol being active analgesics these co-crystals are highly useful in the treatment of pain, especially also not losing any activity/weight by the addition of pharmacologically useless counterions as in salts with no API. In addition the two active principles are complementing each other in the treatment especially of pain, but possibly also of various other diseases or symptoms. Thus, the co-crystals according to the invention do combine a high number of advantages over the state of the art.

Another advantage is that the association of two active principles into one unique species seems to allow for a better Pharmacokinetic/Pharmacodynamic (PKPD) including also a better penetration of the blood-brain barrier, which helps in the treatment of pain.

In general in most embodiments in which the co-crystals of tramadol are used (e.g. for the treatment of pain) these co-crystals would be formulated into a convenient pharmaceutical formulation or a medicament. Accordingly a desirable advantage of a co-crystal of tramadol would show improved pharmaceutical properties and features, especially when compared to the free base or tramadol hydrochloride. Thus, the co-crystal of tramadol according to the invention, should desirably show at least one, preferably more, of the following features:
- to have a very small particle size, e.g. from 300 µm or lower; or
- to be and/or remain essentially free of agglomerates; or
- to be less or not very hygroscopic; or
- to help in formulating controlled release or immediate release formulations; or
- to have a high chemical stability; or
if given to a patient
- to decrease the inter- and intra-subject variability in blood levels; or
- to show a good absorption rate (e.g. increases in plasma levels or AUC); or
- to show a high maximum plasma concentration (e.g. Cₘₐₓ); or
- to show decreased time to peak drug concentrations in plasma (tₘₐₓ); or
- to show changes in half life of the compound (t_{1/2}), in whichever direction this change is preferably directed.

The medicament or pharmaceutical compositions according to the present invention may be in any form suitable for the application to humans and/or animals, preferably humans including infants, children and adults and can be produced by standard procedures known to those skilled in the art. The medicament of the present invention may for example be administered parenterally, including intramuscular, intraperitoneal, or intravenous injection, transmucosal or sublingual application; or orally, including administration as tablets, pellets, granules, capsules, lozenges, aqueous or oily solutions, suspensions, emulsions, sprays or as reconstituted dry powdered form with a liquid medium.

Typically, the medicaments according to the present invention may contain 1-60 % by weight of one or more of the co-crystals as defined herein and 40-99 % by weight of one or more auxiliary substances (additives/excipients).

The compositions of the present invention may also be administered topically or via a suppository.

The daily dosage for humans and animals may vary depending on factors that have their basis in the respective species or other factors, such as age, sex, weight or degree of illness and so forth. The daily dosage for humans preferably is in the range of 5 to 500 milligrams of tramadol to be administered during one or several intakes per day.

A further aspect of the invention relates to the use of a co-crystal according to the invention as described above for the treatment of pain, preferably acute pain, chronic pain, neuropathic pain, hyperalgesia, allodynia or cancer pain, including diabetic neuropathy or osteoarthritis or fibromyalgia. Preferably this use is provided in the form of a medicament or a pharmaceutical composition according to the invention as described above.

Another object of the current invention is a method of treatment of pain, preferably acute pain, chronic pain, neuropathic pain, hyperalgesia, allodynia or cancer pain, including diabetic neuropathy or osteoarthritis or fibromyalgia, by providing to a patient in need thereof a sufficient amount of a co-crystal according to the invention as described above. Preferably the co-crystal according to the invention is provided in physiologically suitable form like e.g. in the form of a medicament or a pharmaceutical composition according to the invention as described above.

The present invention is illustrated below with the help of the following figures and examples. These illustrations are given solely by way of example and do not limit the invention.

### Brief description of the figures :

**Figure 1** **:**
   Differential scanning calorimetry (DSC) analysis of co-crystal tramadol · HCl - paracetamol - H₂O 1 : 1 : 1.
**Figure 2****:**
   Thermogravimetric analysis (TGA) of co-crystal tramadol · HCl - paracetamol - H₂O 1 : 1 : 1.
**Figure 3****:**
   X-ray powder diffraction (XRPD) pattern of co-crystal tramadol · HCl - paracetamol - H₂O 1 : 1 : 1.
**Figure 4****:**
   Unit cell contents of the crystal structure of the co-crystal tramadol · HCl - paracetamol - H₂O 1 : 1 : 1; obtained by single crystal X-ray diffraction analysis (SCXRD) showing four molecules each of tramadol, paracetamol and H₂O in the unit cell (program used: Mercury 1.4.2 - CCDC, Cambridge).

### EXAMPLES

### Example 1: Co-crystal of tramadol · HCl - paracetamol - H₂O 1 : 1 : 1

### Process A:

To an assay tube with magnetic stirring containing tramadol·HCl (87 mg, 0.29 mmol) and paracetamol (44 mg, 0.29 mmol, 1 eq.) was added at room temperature ethyl acetate (0.3 mL) and two water drops. The mixture was stirred 1 h at room temperature. The white solid is filtered with a sinter funnel n°3 and washed with 0.2 mL ethyl acetate. After drying at room temperature under vacuum, co-crystal tramdol·HCl - paracetamol - H₂O 1:1:1 was obtained as a white solid (125 mg, 92% Yield).

### Process B:

To an assay tube with magnetic stirring containing tramadol·HCl (87 mg, 0.29 mmol) and paracetamol (44 mg, 0.29 mmol, 1 eq.) was added at room temperature ACN (0.2 mL) and two water drops (complete dissolution). Ethyl acetate (1 mL) was added slowly and the mixture was seeded with previously obtained crystals of the same form (obtained by Process A) and stirred 1 h at room temperature. The white solid, filtered with a sinter funnel n°3 and dried at room temperature under vacuum, furnished the co-crystal tramadol·HCl - paracetamol - H₂O 1:1:1 as a white solid (97 mg, 71% Yield).

### Process C:

To a 100 mL flask with magnetic stirring containing tramadol·HCl (7.31 g, 24.39 mmol) and paracetamol (3.69 g, 24.39 mmol, 1 eq.) was added at room temperature 25 mL of ethyl acetate. To this suspension, H₂O (2 mL, 111.1 mmol, 4.55 eq.) was added dropwise and the mixture was stirred at room temperature. After the addition the mixture was slightly turbid and, after 10 min stirring, a white solid crystallized. Then, the mixture was stirred 1 h at room temperature.
The white solid is filtered with a sinter funnel n°3 and washed with 3 mL ethyl acetate. After drying at room temperature under vacuum, co-crystal tramadol·HCl - paracetamol - H₂O 1:1:1 was obtained as a white solid (10.25 g, 90% Yield).

### CHARACTERISATION OF THE CO-CRYSTAL:

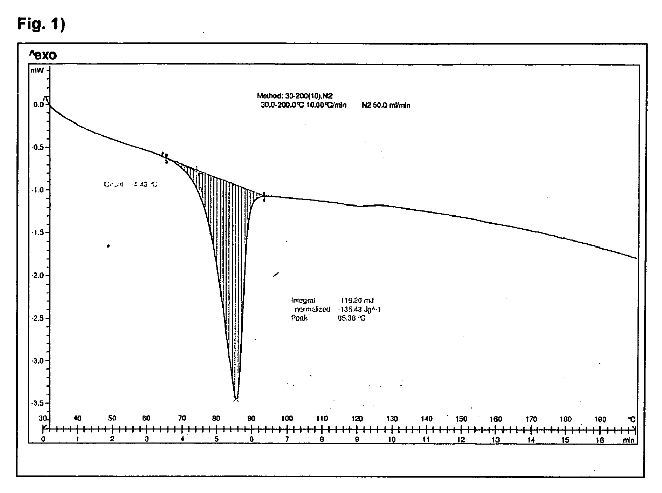

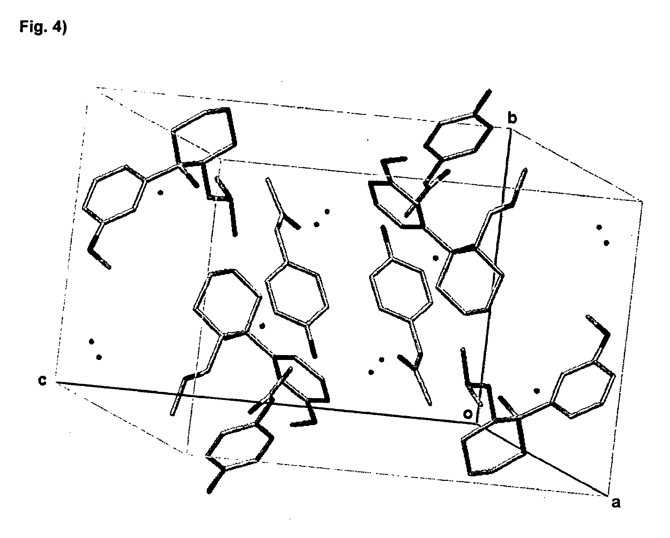

The tramadol·HCl - paracetamol - H₂O (1:1:1) co-crystal obtained according to example 1 was fully characterised by ¹H-NMR, FTIR, X-ray diffraction, Karl-Fisher analysis, DSC and TG (see e.g. figures 1 to 3) and also single crystal X-ray diffraction analysis (see figure 4).

### ¹H NMR

Proton nuclear magnetic resonance analyses were recorded in deuterated dimethylsulfoxide (D6-DMSO) in a Varian Mercury 400 spectrometer, equipped with a broadband probe ATB 1H/19F/X of 5 mm. Spectra were acquired dissolving 5-10 mg of sample in 0.6 mL of deuterated solvent.

¹H NMR spectrum in D6-dimethylsulfoxide at 400 MHz shows peaks at 9.66 (s, 1H) ; 9.55 (s br, 1H) ; 9.12 (s, 1H) ; 7.36-7.28 (m, 2H) ; 7.25 (t, *J* = 8.2 Hz, 3H) ; 7.08-7.03 (m, 2H) ; 6.78 (dd, *J* = 2.3 Hz, *J* = 8.2 Hz, 1H) ; 6.70-6.61 (m, 2H ) ; 5.08 (s, -O*H*, 1H) ; 3.74 (s, O-C*H*₃, 3H) ; 2.89-2.75 (m, 1H) ; 2.63-2.56 (m, N-C*H*₃, 3H) ; 2.46-2.41 (m, N-C*H*₃, 3H) ; 2.42-2.31 (m, 1H) ;2.27-2.15 (m, 1H); 1.95 (s, 3H); 1.94-1.87 (m, 1H); 1.83-1.30 (m, 7H).

### IR

FTIR spectra were recorded using a Thermo Nicolet Nexus 870 FT-IR, equipped with a beamsplitter KBr system, a 35mW He-Ne laser as the excitation source and a DTGS KBr detector. The spectra were acquired in 32 scans at a resolution of 4 cm⁻¹.

The sample (KBr pellets) shows a Fourier Transform Infra Red spectrum with absorption bands at 3356.7 (m), 3302.8 (m), 2937.3 (m), 2678.6 (m br), 1669.18 (s), 1599.0 (m); 1553.8 (m), 1517.5 (s), 1256.7 (s), 1008.1 (m), 837.2 (m), 787.3 (m), 702.5 (m) cm⁻¹

### DSC (Figure 1)

DSC analyses were recorded with a Mettler DSC822^{e}. A sample of 0.8580 mg was weighed into 40 µL aluminium crucible with a pinhole lid and was heated, under nitrogen (50 mL/min), at 10 °C/min from 30 to 200 °C.

The novel type of crystal of the present invention is **characterized in that** the endothermic peak corresponding to the melting point has an onset at 74.43 °C (fusion enthalpy -135.43 J/g), measured by DSC analysis (10 °C/min) (see Figure 1).

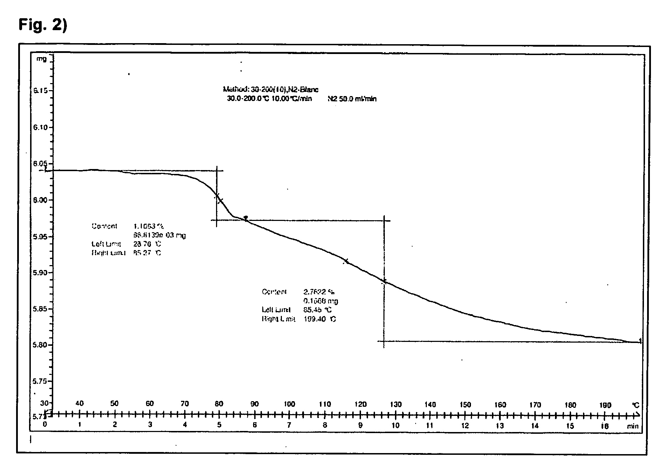

### TGA (Figure 2)

Thermogravimetric analyses were recorded in a thermogravimetric analyzer Mettler TGA/SDTA851^{e}. A sample of 6.0396 mg was weighed into a 70 µL alumina crucible with a pinhole lid and was heated at 10 °C/min from 30 to 200 °C, under nitrogen (50 mL/min).

The TGA analysis of the crystalline form according to the invention shows weight loss 3.86% between 50°C and 200°C: weight of 1 molecule H₂O (see Figure 2).

### Karl Fisher

Karl Fisher analysis was recorded with a Metrohm 787 KF Trinito. Analysis of 194 mg sample was carried out using the following reactants: Hydranal-Composite 5 (Riedel de Haën Ref. 34081), Hydranal Methanol Rapad (Riedel de Haën Ref. 37817) and Hydranal Water Standard 10.0 (Riedel de Haën Ref. 34849 used to calculate the factor).

The Karl Fisher analysis of the crystalline form according to the invention shows 3.8% water as expected for a monohydrate.

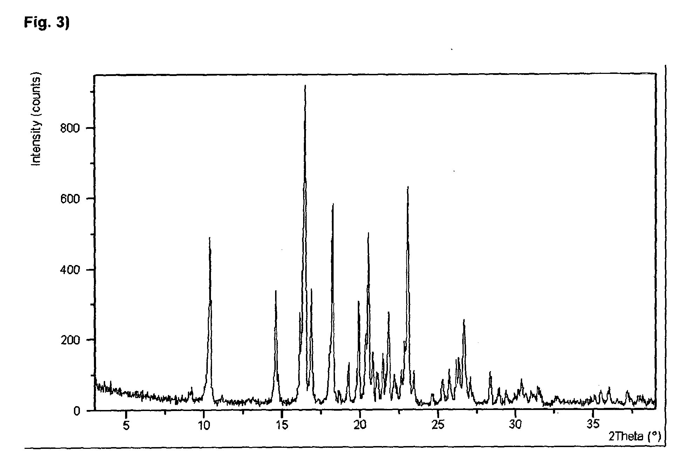

### XRPD: X-ray powder diffraction pattern (Figure 3)

XRPD analysis was performed using a Philips X'Pert diffractometer with Cu K_{α} radiation in Bragg-Brentano geometry. The system is equipped with a proportional detector. The measurement parameters were as follows: the range of 2θ was 3° to 39° at a scan rate of 1.8° per minute.
List of selected peaks, measured on a sample from the evaporation of a solution in acetonitrile, ethyl acetate and water, are as follows (only peaks with relative intensity greater than 1 % are indicated) (see Figure 3):

| **2**θ **(°)** | **d (Å)** | **I (%)** |
|---|---|---|
| 9.18 | 9.63 | 8 |
| 10.38 | 8.52 | 84 |
| 14.59 | 6.07 | 41 |
| 16.13 | 5.50 | 29 |
| 16.48 | 5.38 | 100 |
| 16.88 | 5.25 | 35 |
| 18.22 | 4.87 | 59 |
| 18.65 | 4.76 | 3 |
| 19.24 | 4.61 | 12 |
| 19.90 | 4.46 | 28 |
| 20.33 | 4.37 | 18 |
| 20.54 | 4.32 | 44 |
| 20.81 | 4.27 | 13 |
| 21.11 | 4.21 | 7 |
| 21.46 | 4.14 | 11 |
| 21.82 | 4.07 | 19 |
| 22.22 | 4.00 | 5 |
| 22.64 | 3.93 | 8 |
| 23.06 | 3.86 | 49 |
| 23.46 | 3.79 | 7 |
| 24.62 | 3.62 | 2 |
| 25.28 | 3.52 | 5 |
| 25.71 | 3.47 | 7 |
| 26.18 | 3.40 | 9 |
| 26.35 | 3.38 | 8 |
| 26.65 | 3.34 | 17 |
| 27.07 | 3.29 | 4 |
| 28.36 | 3.15 | 6 |
| 28.91 | 3.09 | 3 |
| 29.36 | 3.04 | 2 |
| 30.37 | 2.94 | 4 |
| 31.01 | 2.88 | 2 |
| 31.40 | 2.85 | 3 |
| 32.65 | 2.74 | 1 |
| 35.09 | 2.56 | 1 |
| 35.43 | 2.53 | 2 |
| 35.98 | 2.50 | 2 |
| 37.15 | 2.42 | 2 |

### SCXRD: Single crystal X-ray diffraction analysis (Figure 4)

Crystal structure has been determined from single crystal X-ray diffraction data. The colourless crystal (0.38 × 0.31 × 0.13 mm) used were obtained from the evaporation of a solution in acetonitrile, ethyl acetate and water of equimolar amounts of tramadol hydrochloride and paracetamol.
Analysis was performed at room temperature using a Bruker Smart Apex diffractometer with graphite monochromated Mo K_{α} radiation equipped with a CCD detector.
Data were collected using phi and omega scans (program used: SMART 5.6). No significant decay of standard intensities was observed. Data reduction (Lorentz and polarization corrections) and absorption correction were applied (program used: SAINT 5.0).
The structure was solved with direct methods and least-squares refinement of Fₒ² against all measured intensities was carried out (program used: SHELXTL -NT 6.1). All non-hydrogen atoms were refined with anisotropic displacement parameters, except oxygen of the disordered water molecule.

### Relevant structural data:

| Crystal system | Monoclinic |
|---|---|
| Space group | *P*2₁/*n* |
| a (Å) | 13.1965(8) |
| b (Å) | 12.1702(8) |
| c (Å) | 17.0828(11) |
| β (°) | 110.631(1) |
| Volume (Å³) | 2567.6(3) |
| Z | 4 |
| D calc. (Mg/m³) | 1.213 |
| N. of refl. | 6260 |
| Refl. with I > 2σ(I) | 4100 |
| R (I > 2σ(I)) | 0.0558 |

The crystal structure is depicted in Figure 4 (hydrogen atoms have been omitted for clarity; the two disordered positions of oxygen of water molecule are shown).
Simulation of XRPD diffractogram from single crystal data gives an almost identical diagram to the experimental one presented above.

## Claims

1. A co-crystal comprising tramadol either as a free base or as its physiologically acceptable salt and paracetamol.

2. The co-crystal according to claim 1, wherein the ratio between tramadol and paracetamol is chosen in such a way that if compared to either tramadol alone or to a mixture of tramadol and paracetamol as active agents
• the solubility of the co-crystal is increased; and/or
• the dose response of the co-crystal is increased; and/or
• the efficacy of the co-crystal is increased; and/or
• the dissolution of the co-crystal is increased; and/or
• the bioavailability of the co-crystal is increased; and/or
• the stability of the co-crystal is increased; and/or
• the hygroscopicity of the co-crystal is decreased; and/or
• the form diversity of the co-crystal is decreased; and/or
• the morphology of the co-crystal is modulated.

3. The co-crystal according to any of claims 1 or 2, wherein the tramadol is racemic tramadol, (-)-tramadol or (+) tramadol.

4. The co-crystal according to any of claims 1 to 3, selected from:
• a co-crystal comprising racemic tramadol either as a free base or as its physiologically acceptable salt and paracetamol;
• a co-crystal comprising (-)-tramadol either as a free base or as its physiologically acceptable salt and paracetamol;
• a co-crystal comprising (+)-tramadol either as a free base or as its physiologically acceptable salt and paracetamol;
• an enantiomeric mixture of co-crystals comprising (-)-tramadol either as a free base or as its physiologically acceptable salt and paracetamol and co-crystals comprising (+)-tramadol either as a free base or as its physiologically acceptable salt and paracetamol; or
• any of the co-crystals above being solvate co-crystals, preferably being hydrate or alcoholate co-crystals, most preferably being monohydrate co-crystals.

5. The co-crystal according to any of claims 1 to 4, wherein the molecular ratio between the tramadol and paracetamol is 1 : 1.

6. The co-crystal according to any of claims 1 to 5, showing a molecular ratio between tramadol, paracetamol and H₂O of 1 : 1 : 1.

7. The co-crystal according to claim 6, **characterized in that** it shows a Fourier Transform Infra Red pattern with absorption bands at 3356 (m), 3302.8 (m), 2937.3 (m), 2678.6 (m br), 1669.18 (s), 1599.0 (m); 1553.8 (m), 1517.5 (s), 1256.7 (s), 1008.1 (m), 837.2 (m), 787.3 (m), and 702.5 (m) cm⁻¹.

8. The co-crystal according to claim 6, **characterized in that** it has a monoclinic unit cell with the following dimensions:
a = 13.20 Å;
b = 12.17 Å;
c = 17.08 Å;
β = 110.63°.

9. A co-crystal according to claim 6, **characterized in that** the endothermic sharp peak corresponding to the melting point has an onset at 74.4 °C.

10. A co-crystal according to claim 6, **characterized in that** it shows an X-ray powder diffreaction pattern with peaks expressed in d-values in A at 8.52, 6.07, 5.50, 5.38, 5.25, 4.87, 4.46, 4.37, 4.32, 4.07, 3.86, and 3.34.

11. Process for the production of a co-crystal according to claim 1 comprising the steps of:
(a) tramadol, optionally in the form of hydrochloride, and paracetamol were added to a container into which also a first non-aqueous solvent is added;
(b) adding water either with, or after, or before adding the solvent from step (a) to the container;
(c) optionally adding a second non-aqueous solvent to the container;
(d) mixing the solution/dispersion of step (b) or optionally step (c) at ambient temperature;
(e) filtering-off the resulting co-crystals.

12. Pharmaceutical composition **characterized in that** it comprises a therapeutically effective amount of the co-crystal according to any of claims 1 to 10 in a physiologically acceptable medium.

13. Use of a co-crystal according to any one of claims 1 to 10 for the treatment of pain, preferably acute pain, chronic pain, neuropathic pain, hyperalgesia, allodynia or cancer pain, including diabetic neuropathy or diabetic peripheral neuropathy and osteoarthritis or fibromyalgia.
